# FASCICULE DE BREVET EUROPEEN

(11) **EP 2 201 103 B1**
(45) Date de publication et mention de la délivrance du brevet: **27.06.2012**
(21) Numéro de dépôt: 08842634.1
(22) Date de dépôt: 10.10.2008
(51) Int. Cl.: C12N 5/071, A61K 39/00, A61K 35/14

(54) **TRAITEMENT DE TUMEURS PAR DES PREPARATIONS DE LYMPHOCYTES T**
BEHANDLUNG VON TUMOREN MIT T-LYMPHOZYT-ZUBEREITUNGEN
TREATMENT OF TUMOURS USING T LYMPHOCYTE PREPARATIONS

(30) Priorité: 12.10.2007 US 960762 P
(43) Date de publication de la demande: 30.06.2010
(73) Titulaire: Université Pierre et Marie Curie (Paris 6), 75005 Paris (FR); Assistance Publique - Hôpitaux de Paris, 75004 Paris (FR); Centre National de la Recherche Scientifique, 75016 Paris (FR); Universite Paris XII, 94010 Creteil (FR)
(72) Inventeur: COHEN, José, F-75020 Paris (FR); KLATZMANN, David, F-75013 Paris (FR); MAURY, Sébastien, F-94340 Joinville Le Pont (FR); LEMOINE, François, F-92120 Montrouge (FR)
(74) Mandataire: Chajmowicz, Marion
(86) Numéro de dépôt international: PCT/FR2008/051842
(87) Numéro de publication internationale: WO 2009/053629

(56) Documents cités:
- DANESE S ET AL: "The janus face of CD4<+>CD25<+> regulatory T cells in cancer and autoimmunity" CURRENT MEDICINAL CHEMISTRY 200703 NL, vol. 14, no. 6, mars 2007 (2007-03), pages 649-666, XP009116113 ISSN: 0929-8673
- CICERI F ET AL: "Antitumor effects of HSV-TK-engineered donor lymphocytes after allogeneic stem-cell transplantation" BLOOD 20070601 US, vol. 109, no. 11, 1 juin 2007 (2007-06-01), pages 4698-4707, XP002526048 ISSN: 0006-4971 0006-4971
- GUILLOT-DELOST MAUDE ET AL: "Clinical-grade preparation of human natural regulatory T-cells encoding the thymidine kinase suicide gene as a safety gene" JOURNAL OF GENE MEDICINE, vol. 10, no. 8, août 2008 (2008-08), pages 834-846, XP002526281 ISSN: 1099-498X(print) 1521-2254(ele
- GIRAUD S ET AL: "Transient depletion of dividing T lymphocytes in mice induces the emergence of regulatory T cells and dominant tolerance to islet allografts" AMERICAN JOURNAL OF TRANSPLANTATION 200805 GB, vol. 8, no. 5, mai 2008 (2008-05), pages 942-953, XP002526049 ISSN: 1600-6135 1600-6143

## Description

La présente invention concerne le domaine de la thérapie cellulaire des cancers. En particulier l'invention a trait au traitement des hémopathies malignes, après rechute ou en première intention.

### ARRIERE-PLAN TECHNOLOGIQUE DE L'INVENTION

L'efficacité de la greffe de cellules souches hématopoïétiques (CSH) allogéniques pour le traitement des hémopathies malignes, dont les leucémies, repose à la fois sur la myéloablation induite par le conditionnement mais aussi sur le transfert, au sein du greffon, de cellules immunocompétentes du donneur exerçant un effet anti-leucémique. Cet effet est dénommé "graft versus leukemia" (GVL) en anglais. L'existence d'un tel effet GVL a été initialement suggérée par des modèles animaux et des analyses statistiques rétrospectives effectuées chez l'homme comparant les taux de rechute de la leucémie entre les patients greffés avec un donneur allogénique ou syngénique, entre ceux recevant un greffon non manipulé ou T-déplété, et entre ceux ayant développé, ou pas, une maladie du greffon contre l'hôte (GVH) aigüe ou chronique (Horowitz, et al. 1990). Plus récemment, la démonstration d'un effet GVL a été apportée par la transfusion de leucocytes provenant du sang périphérique du donneur chez des patients en rechute après la greffe de CSH allogéniques. En effet, chez des patients présentant une rechute de leucémie myéloïde chronique (LMC) après greffe, il a été montré que l'injection de leucocytes provenant du donneur initial (injection de lymphocytes du donneur ou ILD) pouvait induire une nouvelle rémission, sans nécessiter de chimiothérapie ou de radiothérapie complémentaire (Kolb, et al 1990). Cette stratégie est devenue un traitement de première intention chez ces patients et a été développée pour d'autres pathologies hématologiques récidivantes après la greffe telles que les myelodysplasies, les leucémies aigue lymphoblastiques (LAL) et les leucémies aigue myélobastiques (LAM), certains lymphomes, ou les myélomes. Initialement utilisée après greffe avec un donneur familial HLA géno-identique, l'ILD est aujourd'hui aussi utilisée dans le cadre des greffes avec un donneur volontaire de CSH non apparentés (Porter, et al. 2000 ; Dazzi, et al. 2000a).

Chez des patients en rechute après greffe, pour lesquels il y a peu d'alternative, et dont le pronostic est sévère, l'ILD constitue une option thérapeutique acceptable, à laquelle on peut néanmoins espérer apporter des améliorations.
Précédemment, plusieurs stratégies ont été développées dans le but d'amplifier la réponse anti-tumorale après ILD.
La première, développée par l'équipe de S. Slavin, visait à stimuler les Lymphocytes T du donneur soit in vitro avant l'injection soit à la fois in vitro mais aussi in vivo après l'injection, par de l'interleukine 2 (IL-2) recombinante humaine (Slavin, et al, 1996). Une telle injection de Lymphocytes T du donneur « activés » était réservée à des patients ne répondant pas à une injection de Lymphocytes T du donneur frais. Ainsi, chez 5 patients en situation de rechute cytologique de l'hémopathie maligne, l'injection de Lymphocytes T activés a permis d'obtenir une rémission complète de l'hémopathie qui n'avait pas été obtenue après injection de Lymphocytes T du donneur frais. Dans cette étude, il est néanmoins difficile d'attribuer l'efficacité de la seconde injection à l'activation des Lymphocytes T dans la mesure où il peut exister un effet cumulatif bénéfique des injections de Lymphocytes T du donneur, même à l'état frais (Dazzi et al, 2000b). Il faut aussi noter que depuis 1996, cette stratégie d'activation des Lymphocytes T par 1' IL-2 n'a pas fait l'objet de publications venant confirmer son efficacité. De ce fait, son utilisation n'a pas connu de développement à une large échelle.
Une autre stratégie en cours de développement vise à améliorer la présentation des antigènes tumoraux par les cellules leucémiques et les rendre ainsi plus sensibles à l'effet GVL. Elle repose sur des données obtenues in vitro sur des cellules de leucémies myéloïdes, qui peuvent être différenciées en cellules dendritiques à l'aide de cytokines telles que l'interleukine 4 ou le granulocyte monocyte colony stimulating factor (GM-CSF) (Brouwer, et al 2000 ; Chen, et al, 2000). L'utilisation de GM-CSF in vivo est ainsi envisagée pendant la période entourant l'injection de lymphocytes T du donneur afin d'amplifier l'effet GVL attendu (Kolb, et al, 2004).
Une autre stratégie, proposée par Miller et al, 2007 consiste en un conditionnement du patient par une chimiothérapie lymphopéniante, avant une ILD. Ce conditionnement a conduit à une expansion des lymphocytes avec une activation immunitaire accrue.

De leur côté, Powell et al ont essayé d'administrer des lymphocytes autologues déplétés en lymphocytes T régulateurs à des patients atteints d'un mélanome (Powell et al, 2007).

### RESUME DE L'INVENTION

Les inventeurs proposent maintenant d'effectuer une injection de lymphocytes T du donneur, combinée à un conditionnement lymphopéniant, myéloablatif ou non, du patient, dans le but d'augmenter l'effet anti-leucémique des lymphocytes injectés.

Plus précisément l'objet de l'invention est l'utilisation de lymphocytes T, déplétés en lymphocytes T régulateurs, et exprimant une molécule permettant leur destruction spécifique, pour la préparation d'une composition destinée à traiter une tumeur chez un patient, la composition étant destinée à être administrée au patient après un traitement lymphopéniant, myéloablatif ou non, de préférence non-myéloablatif.

Est également décrite une méthode pour traiter une tumeur, la méthode comprenant :
- le conditionnement du patient par un traitement lymphopéniant, de préférence non myéloablatif ;
- l'injection au patient de lymphocytes T, qui ont été préalablement déplétés en lymphocytes T régulateurs, et expriment une molécule permettant leur destruction spécifique.

Cette procédure permet de diminuer le rejet immunitaire contre les lymphocytes T, d'augmenter l'effet anti-leucémique des lymphocytes T injectés, et d'injecter des lymphocytes T provenant d'un fonds génétique différent de celui du premier donneur et du receveur.

Est également décrite une méthode pour traiter une tumeur en première intention, la méthode comprenant :
- le conditionnement du patient par un traitement lymphopéniant, myéloablatif ou non;
- l'injection au patient de cellules souches hématopoïétiques déplétées en lymphocytes T ; et
- l'injection au patient de lymphocytes T, qui ont été préalablement déplétés en lymphocytes T régulateurs, et expriment une molécule permettant leur destruction spécifique.

Selon les cas, on peut en effet utiliser des lymphocytes T autologues ou allogéniques, comme décrit en détails ci-après.

### DESCRIPTION DETAILLEE DE L'INVENTION

### Définitions

Les lymphocytes T sont « déplétés » en lymphocytes T régulateurs, à savoir que la préparation de lymphocytes T administrée au patient ne comprend pratiquement plus de lymphocytes T régulateurs. De préférence, elle comprend moins de 10 % de la fraction des lymphocytes T régulateurs avant déplétion, de préférence encore moins de 1%. de la fraction des lymphocytes T régulateurs avant déplétion.
Les lymphocytes T expriment une « molécule permettant leur destruction spécifique ». Il peut s'agir d'une molécule codée par un transgène ou une molécule exprimée naturellement par les lymphocytes T, lorsque ceux-ci sont allogéniques. Le terme « destruction spécifique » signifie que seuls les lymphocytes T administrés au patient seront détruits, pour empêcher le développement d'une réaction de GVH.
La « molécule permettant leur destruction spécifique » peut être par exemple un antigène du système HLA, les molécules Thy-1, récepteur NGF ou une forme tronquée du récepteur, ou encore un antigène non-immunogène et non exprimé naturellement par les lymphocytes T. Les lymphocytes T portant l'une ou l'autre de ces molécules peuvent alors être détruits spécifiquement par un sérum anti-lymphocytaire, ou des anticorps dirigés spécifiquement contre lesdits antigènes.
La « molécule permettant la destruction spécifique » des lymphocytes T peut également être une molécule codée par un « gène suicide ».
Le terme « gène suicide » se réfère à un gène codant pour une molécule toxique pour la cellule qui l'exprime, de manière conditionnelle.
Les lymphocytes T peuvent être utiles pour traiter le patient après une allogreffe de cellules souches hématopoïetiques, en particulier dans le cas de rechute.
Le terme « rechute » signifie que la tumeur, qui avait montré un régression ou une stagnation, a repris son développement ou, le cas échéant, a métastasé.
Les lymphocytes T peuvent aussi être administrés en première intention, c'est à dire pour traiter des tumeurs qui n'ont pas été soignées auparavant chez le patient par une greffe de cellules souches hématopoïétiques,

### Sources des lymphocytes T

Le donneur est de préférence humain, et peut être un foetus, un nouveau-né, un enfant, un adulte.
Les préparations de lymphocytes T sont obtenues par exemple de sang périphérique, du produit sanguin d'une lymphaphérèse, de ganglions lymphatiques périphériques, de la rate, du thymus, du sang de cordon, etc.
Avant d'être administrés au patient, les lymphocytes T peuvent être manipulés ex vivo de manière à exprimer une molécule permettant leur destruction spécifique, après élimination des lymphocytes T régulateurs. De préférence les lymphocytes Treg sont éliminés avant introduction transduction d'un gène codant pour la molécule permettant la destruction spécifique des lymphocytes T.

Dans le cas d'un traitement d'un patient ayant déjà reçu une greffe de cellules souches hématopoïetiques, les lymphocytes T proviennent de préférence du premier donneur de cellules souches hématopoïetiques. Mais il est également possible que lymphocytes T ne proviennent ni du donneur ni du receveur de la greffe de cellules souches hématopoïétiques.

### Déplétion en lymphocytes T régulateurs (Treg) :

En 1995, le groupe de Shimon Sakaguchi au Japon a identifié les lymphocytes Treg comme constituant une sous-population de lymphocytes T CD4+, représentant environ 5 % d'entre eux, et sont caractérisées par une expression forte et constitutive du marqueur CD25, qui est le récepteur de l'IL-2 (Sakaguchi, et al, 1995).
Des données plus récentes montrent que les Treg humains répriment l'expression du CD 127, qui est le récepteur de l'IL-7 (Seddiki et al, 2006a). Il a également été montré que les Treg avaient un phénotype de cellules naïves CD45RA+ (Seddiki et al, 2006b).

De manière avantageuse, la déplétion en lymphocytes T régulateurs est réalisée ex vivo par sélection négative des cellules CD25+ ou sélection positive des cellules CD127+.
L'élimination des cellules Treg peut être réalisée en utilisant des techniques de séparation par affinité, notamment la séparation magnétique, utilisant des billes magnétiques recouvertes d'anticorps, la chromatographie par affinité, des agents cytotoxiques liés à un anticorps monoclonal ou utilisés en combinaison avec un anticorps monoclonal. Les techniques de séparation peuvent utiliser par exemple des trieurs de cellules actives par fluorescence.

Les réactifs d'affinité peuvent être des récepteurs spécifiques ou des ligands des marqueurs indiqués ci-dessus. De préférence il s'agit d'anticorps, qui peuvent être conjugués par exemple à des billes magnétiques, à de la biotine, ou à des fluorochromes. Des détails sur les techniques possibles de séparation sont présentés dans la demande de brevet US2006/0063256.
Comme dans Attia et al, 2006, qui élimine les lymphocytes T régulateurs sur la base de leur expression du CD25 pour augmenter l'effet antitumoral d'une injection de lymphocytes T dans le cas de cancers solides, on peut d'abord séparer les cellules CD4+, puis parmi celles-ci les cellules CD25+.

Dans un mode de réalisation particulier, les donneurs subissent une cytaphérèse correspondant à deux-trois masses sanguines en utilisant de préférence une machine Cobe Spectra. Cette technique de prélèvement permet d'obtenir en moyenne 1 à 2 x 10¹⁰ cellules mononucléées contenant 20 à 30% de monocytes, 70 à 80 % de lymphocytes T, B et cellules NK. Les cellules sont ensuite déplaquettées et lavées en circuit clos sur machine COBE 2991 permettant ainsi de recueillir une population cellulaire contenant au moins 5 x10⁹ lymphocytes T.
La stratégie de déplétion des lymphocytes Treg est réalisée à partir de cellules provenant soit de produit frais de cytaphérèse ou soit de produit congelé de cytaphérèse notamment lorsque les donneurs n'ont pu être prélevés dans un des centres impliqués dans l'essai. La déplétion des Lymphocytes Treg repose sur l'élimination des cellules CD25-positives. Pour cela un kit de séparation cellulaire en circuit clos Miltényi est utilisé. En bref, les cellules fraîches ou décongelées sont marquées avec un anticorps monoclonal anti-CD25 directement couplé à des billes magnétiques. Les cellules sont ensuite déposées sur colonne de purification et séparées par l'appareil CliniMACS. La fraction positive est enrichie en cellules CD25, la fraction négative est déplétée en cellules CD25. Cette procédure permet de dépléter à plus de 95% les cellules CD25⁺ tout en conservant une population cellulaire contenant plus de 2x10⁹ lymphocytes T. Tandis qu'une fraction des cellules est destinée aux tests de contrôle qualité, à la constitution d'une cellulothèque, la majorité de cellules est remise en culture pour être transduites, amplifiées en culture et sélectionnées avant d'être conditionnée en poches de façon à être injectées aux patients.

### Transduction de molécules permettant la destruction spécifique des lymphocytes T:

Dans un mode de réalisation particulier, les lymphocytes T sont modifiés de manière à exprimer un transgène codant pour une molécule permettant la destruction spécifique desdits lymphocytes T.
De manière préférentielle, le transgène est un gène « suicide ». Par exemple il peut s'agir d'un gène qui code pour une molécule apte à phosphoryler un analogue de nucléoside en une molécule monophosphatée, elle-même convertible par des enzymes cellulaires en nucléotide triphosphaté incorporable dans des acides nucléiques en cours d'élongation sous l'effet des polymérases avec pour effet l'interruption d'élongation des chaînes. Ledit analogue de nucléoside peut être par exemple l'acyclovir ou le gancyclovir. Ladite molécule exprimée par le gène « suicide » peut être notamment la thymidine kinase (TK) du virus de l'herpès simplex de type 1.
La thymidine kinase du virus de l'herpès simplex 1 (HSV1-TK), est apte, lorsqu'elle est présente en une concentration suffisante dans les cellules en cause, à phosphoryler des analogues de nucléosides, tels que l'acyclovir (9-t(2-hydroxyethoxy)méthyl]guanine) ou le gancyclovir (9-[1,3-1Ohydroxy-2- propoxyméthyl]guanine), en des molécules monophosphatées, elles-mêmes convertibles par des enzymes cellulaires en nucléotides triphosphatés incorporables dans des acides nucléiques en cours d'élongation sous l'effet des polymérases au sein desdites cellules, avec pour effet l'interruption d'élongation de chaînes et la mort cellulaire qui s'ensuit.
En cas de GVH, on administre alors au patient l'analogue de nucléoside (par exemple le gancyclovir).
On peut avoir recours à toute technique adéquate pour transférer le transgène, notamment par infection in vitro des cellules correspondantes par une pseudo-particule virale de type Moloney amphotrope. Ces particules virales sont produites par une lignée cellulaire dite de "packaging" que l'on aura construite au préalable. Une lignée de packaging est capable de fabriquer tous les éléments structuraux constituant une particule virale, mais est incapable d'introduire dans des particules virales en voies de maturation les ARNs viraux produits par cette lignée cellulaire. C'est pourquoi, ces lignées dites de packaging fabriquent en permanence des particules virales vides. L'introduction d'une construction génétique appropriée, qui contient l'ADN recombinant tel qu'il a été défini plus haut, permet à ces lignées de packaging d'être introduites dans les particules virales vides réalisant ainsi des pseudo-particules virales. Ces pseudo-particules virales sont capables d'infecter différentes cellules cibles, cellules cibles qui varient selon la lignée de packaging qu'on a utilisée au départ. Par exemple, si cette lignée de packaging est dérivée d'un virus dit de Moloney amphotrope, les particules virales produites infectent parfaitement les cellules hématopoïétiques humaines.
Les techniques classiques pour la production de lignées de cellules transformées par un vecteur rétroviral (voir par exemple Danos et al, 1988 et Markowitz et al, 1988), peuvent être transposées à la production de lymphocytes. De même les techniques de transfert génétique mettant en oeuvre de tels systèmes (Kasid et al, 1990) peuvent être appliquées au transfert, chez l'homme, des cellules telles qu'elles ont été définies plus haut.
Selon un mode particulier de réalisation de l'invention, les lymphocytes T peuvent être modifiés génétiquement en utilisant un vecteur rétroviral SFCMM-2 codant pour le gène « suicide » de fusion HSV-TK/Neo, selon le procédé de transduction décrit dans Ciceri et al, 2007.
Un exemple de protocole de transduction comprend une première phase de culture cellulaire de 1 à 6 jours pour induire l'activation et la mise en cycle des cellules puis une deuxième phase d'infection rétrovirale de 24 heures. L'activation et la mise en cycle des lymphocytes est obtenue en 1 à 6 jours après activation par un anticorps anti-CD3 en milieu RPMI contenant 10% de sérum humain et 600 UI/ml d'interleukine 2 recombinante humaine. La veille de l'infection, le milieu de culture est renouvelé. L'infection proprement dite est réalisée avec du surnageant contenant soit des particules rétrovirales pseudotypées avec une enveloppe provenant du virus de la leucémie du Gibbon (GALV) soit des particules lentivirales pseudotypées avec une enveloppe provenant du virus VSV et contenant le vecteur rétroviral bicistronique Thy-1-IRES-HSV1-TK. Après infection rétrovirale, les cellules sont remises en culture avant d'être sélectionnées.
La sélection des lymphocytes transduits peut être effectuée grâce à la présence du gène rapporteur Thy-1 qui permet de sélectionner les lymphocytes T transduits à l'aide de billes immunomagnétiques. Ainsi, les lymphocytes T sont incubés en présence d'un anticorps monoclonal anti-Thy-1 directement couplé à la biotine. Les cellules sont ensuite incubées avec des microbilles couplées à la Streptavidine puis déposées sur une colonne magnétique. Les cellules transduites Thy-1 positives sont séparées par technique immunomagnétique. Après sélection immunomagnétique, une population de lymphocytes T Thy-1 positifs purifiée au moins 90% est obtenue. Après sélection, les lymphocytes sont soit injectés au receveur soit congelés dans l'attente d'une utilisation ultérieure.

### Validation phénotypique et fonctionnelle des préparations cellulaires:

De préférence, les préparations de lymphocytes T sont testées pour vérifier leur phénotype. Des exemples de tels tests sont décrits ci-après.
a) Analyse immunophénotypique : les populations cellulaires sont étudiées par cytométrie en flux avant et après déplétion en cellules CD25, avant et après modification génétique, avant et après culture, avant et après sélection des cellules transduites. Pour cela, des techniques de double triple voire quadruple marquages des cellules par des anticorps monoclonaux sont utilisées. Les pourcentages de cellules B, NK et monocytes sont déterminées avec les marqueurs CD19, CD14, CD16, CD56, CD45. Les sous-populations de lymphocytes T sont étudiées en utilisant les marqueurs CD3, CD4, CD8, CD45RO, CD45RA, CD62L. Le pourcentage de Lymphocytes Treg est évalué sur le marqueur CD25 et sur le marqueur FOXP3 ou CD127 ou CD45RA seul et en combinaison de marqueurs
b) Analyse fonctionnelle :Les lymphocytes T sont analysés sur le plan fonctionnel avant et après chacune des étapes de manipulation selon deux méthodes :
   - Stimulation allogénique : Les cellules du donneur déplétées ou non en Lymphocytes Treg sont mises en présence de cellules mononucléées irradiées du receveur, les cellules mononucléées irradiées provenant d'un volontaire sont utilisées comme contrôle positif. La prolifération cellulaire dans ces différentes conditions, évaluée par test d'incorporation en thymidine tritiées, permet de comparer si la déplétion en Lymphocytes Treg induit in vitro une augmentation de l'alloréactivité. Dans ces conditions de stimulation allogénique, on peut étudier également par cytométrie de flux la sécrétion intra-cytoplasmique de cytokines (IFN-Gamma, IL-2, IL-4, IL-10) par les lymphocytes T.
   - Réponse aux antigènes de rappel : Les cellules déplétées ou non en Lymphocytes Treg du donneur sont cultivées pendant 4 jours en présence de cellules dendritiques autologues irradiées chargées ou non avec différents antigènes tels que la toxine tétanique, la tuberculine (PPD) et/ ou la candidine. Les cellules dendritiques générées à partir des monocytes du donneur servent de cellules présentatrices. La prolifération lymphocytaire T en réponse à ces antigènes de rappel est évaluée par test d'incorporation de thymidine tritiée. On peut ainsi apprécier in vitro si les populations déplétées en Lymphocytes Treg ont une meilleure capacité ou non à répondre à des antigènes classiques de rappel.

### Patient

Le patient visé est un humain, quel que soit son âge et son sexe. Lorsque le patient est âgé, une greffe de cellules souches hématopoïétiques n'est pas indiquée. On préfèrera alors administrer les lymphocytes T en première intention.
Le patient est atteint d'une tumeur, en particulier d'une tumeur cancéreuse. Il peut s'agir d'une tumeur solide ou d'une hémopathie maligne.
Parmi les tumeurs solides, on peut citer les cancers du poumon, peau, rein, vessie, os, foie, pancréas, ovaires, sein, utérus, prostate, colon, colorectal, tête et cou, etc.
Parmi les hémopathies malignes, on peut citer la leucémie aiguë, la myelodysplasie, ou le syndrome lymphoprolifératif (tel leucémie lymphoïde chronique, myélome, lymphome). les myelodysplasies, les leucémies aigües lymphoblastiques (LAL) et les leucémies aiguës myélobastiques (LAM), des lymphomes, ou un myélome.
Le patient peut notamment souffrir d'une rechute tumorale, par exemple une rechute d'hémopathie maligne.
Dans un mode de réalisation particulier, le patient a subi une allogreffe de cellules souches hématopoïétiques. Dans ce cas, les lymphocytes T administrés peuvent ne provenir ni du donneur ni du receveur de la greffe de cellules souches hématopoïétiques.

De préférence l'allogreffe antérieure de CSH provient d'un donneur familial, de préférence HLA géno-identique, ou d'un donneur volontaire non apparenté. Il peut s'agir d'une greffe à conditionnement myelo-ablatif ou non myelo-ablatif, et elle peut avoir été T-déplétée ou pas.

Le patient visé peut présenter une rechute moléculaire, cytogénétique, ou cytologique de l'hémopathie quelle qu'en soit la date après la greffe.
Les critères de rechute sont définis selon l'hémopathie traitée.
Par exemple, pour une Leucémie aiguë (LAM ou LAL) et myelodysplasie:
- persistance de blastes sanguins et/ou excès de blastes médullaires (>5%), et/ou
- en cas de maladie résiduelle analysable sur le plan moléculaire: absence de diminution (d'au moins un log) du signal moléculaire par rapport au point pré-ILD, ou diminution suivie d'une réascension (d'au moins un log par rapport au nadir).
- en cas de maladie résiduelle analysable sur le plan cytogénétique (classique ou FISH): absence de diminution (d'au moins 50%) du nombre de mitoses portant la(les) anomalie(s), ou diminution suivie d'une réascension (d'au moins 50% par rapport au nadir).
Pour un myélome:
- Stabilité ou augmentation du pic monoclonal par rapport au point pré-ILD.
- Myélome à chaînes légères: Stabilité ou augmentation des paramètres évaluables (lésions osseuses, protéinurie, infiltration médullaire plasmocytaire).
- Absence de diminution, diminution suivi d'une réaugmentation, ou apparition d'une tumeur plasmocytaire.
Pour une leucémie lymphoïde chronique, des lymphomes:
- Stabilité ou augmentation du clone (évalué par cytométrie de flux, biologie moléculaire) par rapport au point pré-ILD.
- Absence de diminution ou diminution suivi d'une réaugmentation du syndrome tumoral (évalué sur le plan clinique et/ou radiologique) par rapport au point pré-ILD.

Lorsqu'ils sont destinés à être administrés au patient en première intention, les lymphocytes T peuvent être autologues vis à vis du patient. Ils expriment alors un transgène, de préférence un gène « suicide », permettant leur destruction spécifique. Les lymphocytes T administrés peuvent aussi être allogéniques. Ils peuvent alors exprimer un transgène permettant leur destruction spécifique, ou peuvent être détruits par un sérum antilymphocytaire.
Lorsqu'il sont destinés à être administrés à un patient ayant déjà subi une allogreffe de cellules souches hématopoïétiques, les lymphocytes T sont allogéniques, ou proviennent de tiers (à savoir ni du patient, ni du donneur de la première greffe). Ils peuvent alors exprimer un transgène permettant leur destruction spécifique, ou peuvent être détruits par un sérum antilymphocytaire

### Conditionnement du patient

Avant l'administration des lymphocytes T, le patient reçoit un traitement lymphopéniant non-myéloablatif.
De préférence le traitement lymphopéniant non myéloablatif est appliqué de 2 à 8 jours avant l'administration des lymphocytes T.
Le patient peut aussi recevoir un traitement lymphopéniant et myéloablatif auquel cas il reçoit en plus une greffe de cellules souches hématopoïétiques plus la préparation de lymphocytes T
Une revue de ce type de traitement est présentée dans Petersen, 2007.
Le patient peut être totalement irradié (« total body irradiation », par exemple à 2Gy), et/ou recevoir une chimiothérapie à base de cyclophosphamide, fludarabine, et/ou endoxan. Par exemple Miller et al, 2007, décrivent un traitement lymphopéniant non myéloablatif, qui comprend une injection IV de cyclophosphamide 50mg/kg une fois à J-6 et J-5, et injection de fludarabine 25mg/m² de J-6 à J-2 (J étant le jour de la greffe des lymphocytes T). Powell et al ; 2007, décrivent un autre protocole comprenant l'injection de fludarabine 25mg/m² pendant 5 jours et d'endoxan 60mg/kg/jours pendant 2 jours.

### Exemple de protocole

Selon un mode de réalisation particulier, la composition à injecter au patient à traiter comprend des lymphocytes T modifiés obtenus par :
i. lymphaphérèse du donneur ;
ii. élimination des lymphocytes T régulateurs
iii. transduction d'un gène codant pour une molécule permettant la destruction spécifique des lymphocytes T,
iv. puis culture des lymphocytes T ainsi modifiés pendant dix à 21 jours.

Plus précisément, le protocole suivant peut être appliqué:
Vers J-15, on prélève des lymphocytes T du donneur et on élimine ex vivo les T régulateurs.
Puis le même jour on active in vitro (par exemple OKT3/IL-2) pendant une durée de quelques heures à 6 jours, puis on infecte les cellules en présence d'un surnageant viral (rétro ou lentiviral) comprenant le gène suicide codant pour la TK et un gène de sélection membranaire, par exemple Thy.1.
Puis on cultive les lymphocytes T jusqu'à l'obtention du nombre de cellules désirées (par exemple 15 jours). Les lymphocytes exprimant le gène TK seront sélectionnés sur la base de l'expression du gène Thy-1 utilisé comme gène rapporteur.
A J-6, on administre le traitement lymphopéniant au patient.
A J0, on injecte les lymphocytes T TK+ Treg déplétés, à des doses comprises entre 2.10⁶ et 10⁸ CD3+/kg.
En cas de GVH, du ganciclovir est administré quelque soit le moment post injection des lymphocytes T du donneur.

### REFERENCES BIBLIOGRAPHIQUES

- Attia P, Powell DJ Jr, Maker AV, Kreitman RJ, Pastan I, Rosenberg SA.: J Immunother (1997). 2006 Mar-Apr;29(2):208-14. Selective élimination of human regulatory T lymphocytes in vitro with the recombinant immunotoxin LMB-2.
- Brouwer, RE, van der Hoorn, M, Kluin-Nelemans, HC, van Zelderen-Bhola, S, Willemze, R, and Falkenburg, JH. 2000. The generation of dendritic-like cells with increased allostimulatory function from acute myeloid leukemia cells of various FAB subclasses. Hum Immunol. 61:565-574.
- Ciceri F, Bonini C, Marktel S, Zappone E, Servida P, Bernardi M, Pescarollo A, Bondanza A, Peccatori J, Rossini S, Magnani Z, Salomoni M, Benati C, Ponzoni M, Callegaro L, Corradini P, Bregni M, Traversari C, Bordignon C. 2007 Blood. 109(11):4698-707. Antitumor effects of HSV-TK-engineered donor lymphocytes after allogeneic stem-cell transplantation.
- Chen, X, Regn, S, Raffegerst, S, Kolb, HJ, and Roskrow, M. 2000. Interferon alpha in combination with GM-CSF induces the differentiation of leukaemic antigen-presenting cells that have the capacity to stimulate a specific anti-leukaemic cytotoxic T-cell response from patients with chronic myeloid leukaemia. Br J Haematol. 111:596-607.
- Danos, O. and Mulligan R. C. 1988. Safe and efficient generation of recombinant retroviruses with amphotropic and ecotropic host ranges. Proc. Natl. Acad. Sci. USA 85, 6460-6464.
- Dazzi, F, Szydlo, RM, Cross, NC, Craddock, C, Kaeda, J, Kanfer, E, Cwynarski, K, Olavarria, E, Yong, A, Apperley, JF, et al. 2000a. Durability of responses following donor lymphocyte infusions for patients who relapse after allogeneic stem cell transplantation for chronic myeloid leukemia. Blood. 96:2712-2716.
- Dazzi, F, Szydlo, RM, Craddock, C, Cross, NC, Kaeda, J, Chase, A, Olavarria, E, van Rhee, F, Kanfer, E, Apperley, JF, et al. 2000b. Comparison of single-dose and escalating-dose regimens of donor lymphocyte infusion for relapse after allografting for chronic myeloid leukemia. Blood. 95:67-71.
- Horowitz, MM, Gale, RP, Sondel, PM, Goldman, JM, Kersey, J, Kolb, HJ, Rimm, AA, Ringden, O, Rozman, C, Truitt, RL, et al. 1990. Graft-versus-leukemia reactions after bone marrow transplantation. Blood. 75:555-562.
- Kasid, A., Morecki, S., Aebersold, P., Cornetta, K., Culver, K., Freeman, S., Director, E. Lotze, M. T., Blaese, R. M. Anderson., W. F., and Rosenberg, S. A. 1990. Human gene transfer: characterization of human tumor-infiltrating lymphocytes as vehicles for retroviral-mediated gene transfer in man. Proc. Natl. Sci. USA 87, 473-477.
- Kolb, HJ, Mittermuller, J, Clemm, C, Holler, E, Ledderose, G, Brehm, G, Heim, M, and Wilmanns, W. 1990. Donor leukocyte transfusions for treatment of recurrent chronic myelogenous leukemia in marrow transplant patients. Blood. 76:2462-2465.
- Kolb, HJ, Schmid, C, Barrett, AJ, and Schendel, DJ. 2004. Graft-versus-leukemia reactions in allogeneic chimeras. Blood. 103:767-776.
- Miller JS, Weisdorf DJ, Burns LJ, Slungaard A, Wagner JE, Verneris MR, Cooley S, Wangen R, Fautsch SK, Nicklow R, Defor T, Blazar BR. 2007 Blood.110(7):2761-3, Lymphodepletion followed by donor lymphocyte infusion (DLI) causes significantly more acute graft-versus-host disease than DLI alone.
- Markowitz, D., Goff, S., and Bank, A. 1988. Construction and use of a safe and efficient amphotropic packaging cell line. Virology 167:400-406.
- Petersen 2007. Medical Bulletin, 54 :112-39.Alloreactivity of therapeutic principle in the treatment of hematologic malignancies.
- Porter, DL, Collins, RH, Jr., Hardy, C, Keman, NA, Drobyski, WR, Giralt, S, Flowers, ME, Casper, J, Leahey, A, Parker, P, et al. 2000. Treatment of relapsed leukemia after unrelated donor marrow transplantation with unrelated donor leukocyte infusions. Blood. 95:1214-1221.
- Powell DJ Jr, de Vries CR, Allen T, Ahmadzadeh M, Rosenberg SA. 2007 Inability to mediate prolonged reduction of regulatory T Cells after transfer of autologous CD25-depleted PBMC and interleukin-2 after lymphodepleting chemotherapy. J Immunother May-Jun;30(4):438-47.
- Sakaguchi, S, Sakaguchi, N, Asano, M, Itoh, M, and Toda, M. 1995. Immunologic self-tolerance maintained by activated T cells expressing IL-2 receptor alpha-chains (CD25). Breakdown of a single mechanism of self-tolerance causes various autoimmune diseases. J Immunol. 155:1151-1164.
- Seddiki N, Santner-Nanan B, Martinson J, Zaunders J, Sasson S, Landay A, Solomon M, Selby W, Alexander SI, Nanan R, Kelleher A, Fazekas de St Groth B.2006a. Expression of interleukin (IL)-2 and IL-7 receptors discriminates between human regulatory and activated T cells. J Exp Med. Jul 10;203(7):1693-700.
- Seddiki N, Santner-Nanan B, Tangye SG, Alexander SI, Solomon M, Lee S, Nanan R, Fazekas de Saint Groth B.2006b. Persistence of naive CD45RA+ regulatory T cells in adult life.Blood.107(7):2830-8.
- Slavin, S, Naparstek, E, Nagler, A, Ackerstein, A, Samuel, S, Kapelushnik, J, Brautbar, C, and Or, R. 1996. Allogeneic cell therapy with donor peripheral blood cells and recombinant human interleukin-2 to treat leukemia relapse after allogeneic bone marrow transplantation. Blood. 87:2195-2204.

## Revendications

1. Composition de lymphocytes T, déplétés en lymphocytes T régulateurs, et exprimant une molécule permettant leur destruction spécifique, pour une utilisation dans le traitement d'une tumeur chez un patient, la composition étant destinée à être administrée au patient après un traitement lymphopéniant.

2. Composition selon la revendication 1, dans laquelle la tumeur est une hémopathie maligne, telle qu'une leucémie aigüe, une myélodysplasie ou un syndrome lymphoprolifératif.

3. Composition selon la revendication 1, dans laquelle la tumeur est une tumeur solide.

4. Composition selon la revendication 1, dans laquelle les lymphocytes T expriment un gène « suicide » permettant leur destruction spécifique.

5. Composition selon la revendication 4, dans laquelle le gène dit « suicide » code pour une molécule capable de réagir avec un analogue de nucléoside pour conduire à la mort desdits lymphocytes T, ladite molécule codée par le gène « suicide » étant de préférence une molécule apte à phosphoryler un analogue de nucléoside en une molécule monophosphatée, elle-même convertible par des enzymes cellulaires en nucléotide triphosphaté incorporable dans des acides nucléiques en cours d'élongation sous l'effet des polymérases avec pour effet l'interruption d'élongation des chaînes.

6. Composition selon la revendication 5, dans laquelle ledit analogue de nucléoside est l'acyclovir ou le gancyclovir.

7. Composition selon la revendication 5, dans laquelle ladite molécule codée par le gène « suicide » est la thymidine kinase du virus de l'herpès simplex de type 1.

8. Composition selon la revendication 1, dans laquelle le patient souffre d'une rechute cancéreuse, après une allogreffe de cellules souches hématopoïétiques.

9. Composition selon la revendication 8, dans laquelle lesdits lymphocytes T ne proviennent ni du donneur ni du receveur de l'allogreffe de cellules souches hématopoïétiques.

10. Composition selon la revendication 1, dans laquelle les lymphocytes T sont destinés à être administrés au patient en première intention, ledit patient n'ayant pas subi de greffe de cellules souches hématopoïétiques et dans laquelle les lymphocytes T sont de préférence autologues vis à vis du patient et expriment un transgène permettant leur destruction spécifique.

11. Composition l'une des revendications 1 à 7, dans laquelle les lymphocytes T administrés au patient sont des lymphocytes T allogéniques.

12. Composition selon l'une des revendications 1 à 11, dans laquelle la déplétion en lymphocytes T régulateurs est réalisée ex vivo par sélection négative des cellules CD25+ ou sélection positive des cellules CD127+.

13. Composition selon l'une des revendications 1 à 12, dans laquelle le traitement lymphopéniant est non myéloablatif et consiste, de préférence, en une administration de cyclophosphamide, fludarabine et/ou endoxan.

14. Composition selon l'une des revendications 1 à 13, dans laquelle la composition est destinée à être administrée de 2 à 8 jours après le traitement lymphopéniant non myéloablatif.

15. Composition selon l'une des revendications 1 à 14, dans laquelle la composition comprend des lymphocytes T modifiés obtenus par :
i. lymphaphérèse du donneur ;
ii. élimination des lymphocytes T régulateurs
iii. transduction d'un gène codant pour une molécule permettant la destruction spécifique des lymphocytes T,
iv. puis culture des lymphocytes T ainsi modifiés pendant 10 à 21 jours.

## Claims

1. Composition of T lymphocytes, depleted of regulatory T lymphocytes, and expressing a molecule allowing their specific destruction, for use in the treatment of a tumor in a patient, the composition being administered to the patient after a lymphopenic treatment.

2. The composition according to Claim 1, wherein the tumor is a malignant haemopathy, as an acute leukaemia, a myelodysplasia or a lymphroproliferative syndrome.

3. The composition according to Claim 1, wherein the tumor is a solid tumor.

4. The composition according to Claim 1, wherein the T lymphocytes express a "suicide" gene allowing their specific destruction.

5. The composition according to Claim 4, in which said "suicide" gene encodes a molecule capable of reacting with a nucleoside analogue in order to lead to the death of the said T lymphocytes, said molecule encoded by a "suicide" gene being preferably a molecule capable of phosphorylating a nucleoside analogue to a monophosphate molecule, itself convertible by cellular enzymes to a triphosphate nucleotide that can be incorporated into nucleic acids during extension under the effect of polymerases, the effect being the interruption of chain extension.

6. The composition according to Claim 5, wherein said nucleoside analogue is acyclovir or gancyclovir.

7. The composition according to Claim 5, wherein said molecule encoded by the "suicide" gene is thymidine kinase of the herpes simplex virus type 1.

8. The composition according to Claim 1, wherein the patient is suffering from a cancer relapse, following an allotransplantation of haematopoietic stem cells.

9. The composition according to Claim 8, wherein said T lymphocytes are obtained from neither the donor nor the recipient of the haematopoietic stem cell allograft.

10. The composition according to Claim 1, wherein the T lymphocytes are intended to be administered to the patient as a first line, the said patient not having undergone the transplantation of haematopoietic stem cells and in which the T lymphocytes are preferably autologous with respect to the patient and express a transgene allowing their specific destruction.

11. The composition according to Claims 1 to 7, wherein the T lymphocytes administered to the patent are allogenic T lymphocytes.

12. The composition according to Claims 1 to 11, wherein the depletion of regulatory T lymphocytes is performed ex vivo by negative selection of the CD25+ cells or positive selection of the CD127+ cells.

13. The composition according to Claims 1 to 12, wherein the lymphopenic treatment is non-myeloablative and consists, preferably, of an administration of cyclophosphamide, fludarabine and/or endoxan.

14. The composition according to Claims 1 to 13, wherein the composition is to be administered 2 to 8 days after the non-myeloablative lymphopenic treatment.

15. The composition according to Claims 1 to 14, wherein the composition comprises modified T lymphocytes obtained by:
i. lymphapheresis of the donor;
ii. removal of the regulatory T lymphocytes
iii. transduction of a gene encoding a molecule allowing the specific destruction of the T lymphocytes,
iv. and then culture of the T lymphocytes thus modified for 10 to 21 days.

## Patentansprüche

1. Zusammensetzung von T-Lymphozyten, die an regulatorischen T-Lymphozyten depletiert sind und ein Molekül exprimieren, das ihre spezifische Zerstörung erlaubt, für eine Verwendung in der Behandlung eines Tumors bei einem Patienten, wobei die Zusammensetzung dem Patienten nach einer lymphopenischen Behandlung verabreicht werden soll.

2. Zusammensetzung gemäß Anspruch 1, wobei der Tumor eine maligne Hämopathie, wie eine akute Leukämie, eine Myelodysplasie oder ein lymphoproliferatives Syndrom, ist.

3. Zusammensetzung gemäß Anspruch 1, wobei der Tumor ein solider Tumor ist.

4. Zusammensetzung gemäß Anspruch 1, wobei die T-Lymphozyten ein "Suizid"-Gen exprimieren, das ihre spezifische Zerstörung erlaubt.

5. Zusammensetzung gemäß Anspruch 4, wobei das als "Suizid"-Gen bezeichnete Gen für ein Molekül codiert, das in der Lage ist, mit einem Nukleosid-Analogon zu reagieren, um zum Absterben besagter T-Lymphozyten zu führen, wobei besagtes von dem "Suizid"-Gen codiertes Molekül vorzugsweise ein Molekül ist, das in der Lage ist, ein Nukleosid-Analogon zu einem Monophosphat-Molekül zu phosphorylieren, das wiederum selbst von Zellenzymen in ein Nukleotidtriphosphat umwandelbar ist, das in Nukleinsäuren bei der Elongation durch die Wirkung der Polymerasen eingebaut werden kann, mit der Folge den Abbruch der Elongation der Ketten zu bewirken.

6. Zusammensetzung gemäß Anspruch 5, wobei besagtes Nukleosid-Analogon Acyclovir oder Gancyclovir ist.

7. Zusammensetzung gemäß Anspruch 5, wobei besagtes Molekül, das von dem "Suizid"-Gen codiert wird, die Thymidin-Kinase des Herpes-simplex-Virus Typ 1 ist.

8. Zusammensetzung gemäß Anspruch 1, wobei der Patient an einem Krebs-Rezidiv nach einer Allotransplantation von hämatopoetischen Stammzellen leidet.

9. Zusammensetzung gemäß Anspruch 8, wobei besagte T-Lymphozyten weder vom Spender noch vom Empfänger des hämatopoetischen Stammzelltransplantats stammen.

10. Zusammensetzung gemäß Anspruch 1, wobei die T-Lymphozyten dem Patienten in einer Erstbehandlung verabreicht werden sollen, wobei besagter Patient keiner Transplantation von hämatopoetischen Stammzellen unterzogen worden ist und wobei vorzugsweise die T-Lymphozyten bezogen auf den Patienten autolog sind und ein Transgen exprimieren, das ihre spezifische Zerstörung erlaubt.

11. Zusammensetzung gemäß einem der Ansprüche 1 bis 7, wobei die dem Patienten verabreichten T-Lymphozyten allogene T-Lymphozyten sind.

12. Zusammensetzung gemäß einem der Ansprüche 1 bis 11, wobei die Depletion an regulatorischen T-Lymphozyten durch negative Selektion der CD25+-Zellen oder positive Selektion der CD127+-Zellen ex vivo erfolgt.

13. Zusammensetzung gemäß einem der Ansprüche 1 bis 12, wobei die lymphopenische Behandlung nicht myeloablativ ist und vorzugsweise aus einer Verabreichung von Cyclophosphamid, Fludarabin und/oder Endoxan besteht.

14. Zusammensetzung gemäß einem der Ansprüche 1 bis 13, wobei die Zusammensetzung 2 bis 8 Tage nach der nicht-myeloablativen lymphopenischen Behandlung verabreicht werden soll.

15. Zusammensetzung gemäß einem der Ansprüche 1 bis 14, wobei die Zusammensetzung modifizierte T-Lymphozyten umfasst, die gewonnen werden mittels:
i. Lymphapherese des Spenders;
ii. Entfernen der regulatorischen T-Lymphozyten
iii. Transduktion eines Gens, das für ein Molekül codiert, das die spezifische Zerstörung der T-Lymphozyten erlaubt,
iv. anschließender Kultivierung der auf diese Weise modifizierten T-Lymphozyten für 10 bis 21 Tage.
